(19) Europäisches Patentamt / European Patent Office / Office européen des brevets

(11) EP 2 310 110 B1

(12) **FASCICULE DE BREVET EUROPEEN**

(45) Date de publication et mention de la délivrance du brevet:
**09.09.2015 Bulletin 2015/37**

(21) Numéro de dépôt: **09784286.8**

(22) Date de dépôt: **21.07.2009**

(51) Int Cl.:
***B01D 53/14*** *(2006.01)* ***C07C 209/00*** *(2006.01)*
***C07C 213/00*** *(2006.01)* ***B01D 53/62*** *(2006.01)*
***B01D 53/78*** *(2006.01)*

(86) Numéro de dépôt international:
**PCT/FR2009/000901**

(87) Numéro de publication internationale:
**WO 2010/012883 (04.02.2010 Gazette 2010/05)**

(54) **SOLUTION ABSORBANTE A BASE DE N,N,N',N'-TETRAMETHYLHEXANE-1,6-DIAMINE ET D'UNE AMINE COMPORTANT DES FONCTIONS AMINE PRIMAIRE OU SECONDAIRE ET PROCEDE D'ELIMINATION DE COMPOSES ACIDES D'UN EFFLUENT GAZEUX**

ABSORBIERENDE LÖSUNG AUF DER BASIS VON N,N,N',N'-TETRAMETHYLHEXAN-1,6-DIAMIN UND EINEM AMIN MIT PRIMÄREN ODER SEKUNDÄREN AMINGRUPPEN UND VERFAHREN ZUR ELIMINIERUNG VON SAUREN KOMPONENTEN AUS EINEM GASSTROM

ABSORBENT SOLUTION BASED ON N,N,N',N'-TETRAMETHYLHEXANE-1,6-DIAMINE AND ON ONE PARTICULAR AMINE COMPRISING PRIMARY OR SECONDARY AMINE FUNCTIONAL GROUPS AND PROCESS FOR REMOVING ACID COMPOUNDS FROM A GASEOUS EFFLUENT

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **28.07.2008 FR 0804304**

(43) Date de publication de la demande:
**20.04.2011 Bulletin 2011/16**

(73) Titulaire: **IFP Energies nouvelles**
**92852 Rueil-Malmaison Cedex (FR)**

(72) Inventeur: **JACQUIN, Marc**
**F-69002 Lyon (FR)**

(56) Documents cités:
**EP-A- 0 365 850** **EP-A- 1 775 633**
**FR-A- 2 898 284** **FR-A- 2 900 841**
**US-A1- 2006 011 512**

- **A. KOHL, R. NIELSEN: "Gas Purification, Fifth Edition, Chapter 14 : Mixed Physical/Chemical Solvent Processes" 1997, GULF PUBLISHING COMPANY , HOUSTON, TEXAS , XP002518910 page 1225, alinéa 2-4 page 1231, alinéa 1**

Remarques:
Le dossier contient des informations techniques présentées postérieurement au dépôt de la demande et ne figurant pas dans le présent fascicule.

Il est rappelé que: Dans un délai de neuf mois à compter de la publication de la mention de la délivrance du brevet européen au Bulletin européen des brevets, toute personne peut faire opposition à ce brevet auprès de l'Office européen des brevets, conformément au règlement d'exécution. L'opposition n'est réputée formée qu'après le paiement de la taxe d'opposition. (Art. 99(1) Convention sur le brevet européen).

EP 2 310 110 B1

## Description

### Domaine de l'invention

La présente invention concerne l'absorption de composés acides ($H_2S$, $CO_2$, COS, $CS_2$, mercaptans, ...) contenus dans un gaz, au moyen d'une solution aqueuse absorbante comprenant la combinaison d'une diamine tertiaire particulière, la N,N,N',N'-tétraméthylhexane-1,6-diamine, et d'une amine primaire ou secondaire particulière, permettant d'obtenir une solution absorbante monophasique dans les conditions d'absorption des gaz acides tels que le $CO_2$. L'invention s'applique avantageusement au traitement du gaz naturel et de gaz d'origine industrielle.

### Art antérieur

Traitement des gaz d'origine industrielle

La nature des effluents gazeux que l'on peut traiter est diverse, on peut citer de façon non limitative les gaz de synthèse, les fumées de combustion, les gaz de raffinerie, les gaz obtenus en queue du procédé Claus, les gaz de fermentation de biomasse, les gaz de cimenterie et les gaz de hauts-fourneaux.

Tous ces gaz contiennent des composés acides tels que le dioxyde de carbone ($CO_2$) l'hydrogène sulfuré ($H_2S$), l'oxysulfure de carbone (COS), le disulfure de carbone ($CS_2$) et les mercaptans (RSH), principalement le méthylmercaptan ($CH_3SH$), l'éthylmercaptan ($CH_3CH_2SH$) et les propylmercaptans ($CH_3CH_2CH_2SH$).

Par exemple, dans le cas des fumées de combustion, le $CO_2$ est le composé acide que l'on cherche à éliminer. En effet, le dioxyde de carbone est un des gaz à effet de serre largement produits par différentes activités de l'homme et a un impact direct sur la pollution atmosphérique. Afin de diminuer les quantités de dioxyde de carbone émises dans l'atmosphère, il est possible de capter le $CO_2$ contenu dans un effluent gazeux.

Traitement de gaz naturel

Dans le cas du gaz naturel, trois principales opérations de traitement sont considérées : la désacidification, la déshydratation et le dégazolinage. La première étape, qui est la désacidification, a pour objectif l'élimination des composés acides tels que le dioxyde de carbone ($CO_2$), mais aussi l'hydrogène sulfuré ($H_2S$), l'oxysulfure de carbone (COS), le disulfure de carbone ($CS_2$) et les mercaptans (RSH), principalement le méthylmercaptan ($CH_3SH$), l'éthylmercaptan ($CH_3CH_2SH$) et les propylmercaptans ($CH_3CH_2CH_2SH$). Les spécifications généralement admises sur le gaz désacidifié sont 2% de $CO_2$, voir 50 ppm de $CO_2$ pour réaliser ensuite une liquéfaction du gaz naturel ; 4 ppm d'$H_2S$, et 10 à 50 ppm volume de soufre total. L'étape de déshydratation permet ensuite de contrôler la teneur en eau du gaz désacidifié par rapport à des spécifications de transport. Enfin, l'étape de dégazolinage du gaz naturel permet de garantir le point de rosée des hydrocarbures dans le gaz naturel, là encore en fonction de spécifications de transport.

La désacidification est donc souvent réalisée en premier lieu, notamment afin d'éliminer les gaz acides toxiques tel que l'$H_2S$ dans la première étape de la chaîne de procédés et afin d'éviter la pollution des différentes opérations unitaires par ces composés acides, notamment la section de déshydratation et la section de condensation et de séparation des hydrocarbures les plus lourds.

Élimination des composés acides par absorption

La désacidification des effluents gazeux, tels que par exemple le gaz naturel et les fumées de combustion, ainsi que les gaz de synthèse, les gaz de raffinerie, les gaz obtenus en queue du procédé Claus, les gaz de fermentation de biomasse, les gaz de cimenterie et les gaz de hauts-fourneaux, est généralement réalisée par lavage par une solution absorbante. La solution absorbante permet d'absorber les composés acides présents dans l'effluent gazeux (notamment $H_2S$, mercaptans, $CO_2$, COS, $CS_2$).

Les solvants couramment utilisés aujourd'hui sont les solutions aqueuses d'alcanolamine primaire, secondaire ou tertiaire, en association avec un éventuel solvant physique. On peut citer par exemple le document FR 2 820 430 qui propose des procédés de désacidification d'effluents gazeux. On peut aussi citer par exemple le brevet US 6 852 144 qui décrit une méthode d'élimination des composés acides des hydrocarbures. La méthode utilise une solution absorbante eau-méthyldiéthanolamine ou eau-triéthanolamine contenant une forte proportion d'un composé appartenant au groupe suivant : piperazine et/ou méthylpiperazine et/ou morpholine.

Par exemple, dans le cas du captage du $CO_2$, le $CO_2$ absorbé réagit avec l'amine présente en solution selon une réaction exothermique réversible, bien connue de l'homme du métier et conduisant à la formation d'hydrogénocarbonates, de carbonates et/ou de carbamates, permettant une élimination du $CO_2$ dans le gaz à traiter. De même, pour l'élimination de l'$H_2S$ dans le gaz à traiter, l'$H_2S$ absorbé réagit avec l'amine présente en solution selon une réaction

exothermique réversible, bien connue de l'homme du métier et conduisant à la formation d'hydrogénosulfure.

Un autre aspect primordial des opérations de traitement de gaz ou fumées industrielles par solvant est l'étape de régénération de l'agent de séparation. En fonction du type d'absorption (physique et/ou chimique), on envisage généralement une régénération par détente, et/ou par distillation et/ou par entraînement par un gaz vaporisé appelé "gaz de strippage".

Une des principales limitations des solvants couramment utilisés aujourd'hui est la nécessité de mettre en oeuvre des débits de solution absorbante importants, ce qui entraîne une consommation énergétique importante pour la régénération du solvant, mais également des tailles d'équipement importantes (colonnes, pompes, etc.). Ceci est particulièrement vrai dans le cas où la pression partielle de gaz acides est faible. Par exemple, pour une solution aqueuse de MonoEthanolAmine à 30% poids utilisée pour le captage du $CO_2$ en post-combustion dans une fumée de centrale thermique, où la pression partielle de $CO_2$ est de l'ordre de 0,1 bar, l'énergie de régénération représente environ 3,9 GJ par tonne de $CO_2$ captée (cas de référence, projet CASTOR, pilote de captage en post-combustion de la centrale thermique d'Esbjerg). Une telle consommation énergétique représente un coût opératoire considérable pour le procédé de captage du $CO_2$.

D'une manière générale, pour le traitement d'effluents acides comprenant des composés acides comme par exemple $H_2S$, mercaptans, $CO_2$, COS, $SO_2$, $CS_2$, l'utilisation de composés à base d'amines est intéressante, en raison de leur facilité de mise en oeuvre en solution aqueuse. Cependant, lors de la désacidification de ces effluents, la solution absorbante peut se dégrader, soit par dégradation thermique, soit par réaction secondaire avec les gaz acides à capter, mais aussi avec d'autres composés contenus dans l'effluent, comme par exemple l'oxygène, les SOx et les NOx, contenus dans les fumées industrielles. Ces réactions de dégradation nuisent au bon fonctionnement du procédé : diminution de l'efficacité du solvant, corrosion, moussage, etc. Du fait de ces dégradations, il est nécessaire de réaliser une purification du solvant par distillation et/ou échange d'ions, et réaliser des appoints d'amines. A titre d'exemple, les appoints d'amine dans un procédé de captage du $CO_2$ en post-combustion, utilisant une solution absorbante de MonoEthanolAmine à 30% poids représentent 1,4 kg d'amine par tonne de $CO_2$ capté, ce qui augmente considérablement les coûts opératoires d'une unité de captage.

Enfin, ces réactions de dégradation limitent les conditions de fonctionnement du procédé, notamment la température à laquelle est réalisée la régénération du solvant. A titre d'exemple, augmenter la température du régénérateur de 10°C multiplie par deux la vitesse de dégradation thermique de la MonoEthanolAmine. La régénération des solutions aqueuses d'alcanolamines, comme la MonoEthanolAmine est donc réalisée à des températures en fond de régénérateur de l'ordre de 110°C, voir 130°C pour les amines plus stables, comme la MethylDiEthanolAmine. Du fait de ces températures en fond de régénérate.ur, les gaz acides ($CO_2$, $H_2S$, COS, $CS_2$, etc.) sont obtenus à des pressions modérées, de 1 à 3 bars. Suivant la nature du gaz acide régénéré et les applications, le gaz acide peut être envoyé vers une unité de traitement, ou bien comprimé pour être réinjecté et séquestré.

Il est difficile de trouver un composé absorbant stable, permettant d'éliminer les composés acides dans tout type d'effluent, et permettant au procédé de désacidification de fonctionner à moindre coût. La demanderesse a découvert que la N,N,N',N'-TetraMethylHexane-1,6-D!Amine ou TMHDA, seule ou en mélange avec quelques % poids d'amines primaires ou secondaires, présente un intérêt important dans l'ensemble des procédés de traitement d'effluents gazeux pour l'élimination de composés acides.

Les brevets FR 2 898 284 et FR 2 900 841 décrivent la mise en oeuvre de solutions absorbantes pouvant comprendre de la TMHDA pour la désacidification de gaz.

Néanmoins, la plupart des solutions aqueuses absorbante comprenant la N,N,N',N'-TetraMethylHexane-1,6-DiAmine ou TMHDA, seule ou en mélange avec quelques % poids d'amines primaires ou secondaires, présentent une séparation de phase liquide-liquide lors de l'absorption du $CO_2$ dans les conditions de l'absorbeur. Sous forme de deux phases séparées, le flux de composés acides transférés du gaz vers la solution absorbante serait fortement impacté et la hauteur de colonne devrait être adapté en conséquence. Ce phénomène pose donc de graves problèmes de mise en oeuvre, et compte tenu de la complexité du système, est difficile à modéliser. De manière surprenante, la demanderesse a découvert que l'ajout de quelques % poids d'amines primaires ou secondaires particulières à une solution aqueuse de N,N,N',N'-TetraMethylHexane-1,6-DiAmine permet d'obtenir une solution absorbante monophasique dans les conditions d'absorption des gaz acides tels que le $CO_2$.

## Description de l'invention

La présente invention a donc pour objet de pallier un ou plusieurs des inconvénients de l'art antérieur en proposant une méthode pour éliminer les composés acides, tels que le $CO_2$, l'$H_2S$, le COS, le $CS_2$ et les mercaptans, d'un gaz par l'utilisation de la combinaison de deux amines spécifiques dont les propriétés permettent, tout en conservant une solution absorbante monophasique dans les conditions d'absorption des gaz acides, de limiter le débit de solution absorbante à utiliser, notamment à faible pression partielle de gaz acide, et qui présentent une très grande stabilité.

Un premier objet de l'invention est une solution aqueuse absorbante comprenant la combinaison de la N,N,N',N'-Tétraméthylhexane-1,6-diamine de formule (I) et d'un activateur de formule (II) ou (III).

La présente invention concerne également un procédé d'élimination des composés acides contenus dans un effluent gazeux, tels que le gaz naturel et les gaz d'origine industrielle, comprenant :

- une étape d'absorption des composés acides par mise en contact de l'effluent avec une solution aqueuse comprenant la N,N,N',N'-tétraméthylhexane-1,6-diamine et d'un activateur de formule (II) ou (III).
- éventuellement au moins une étape de séparation liquide-liquide de la solution chargée en gaz acide après chauffage, permettant une régénération fractionnée de la solution absorbante.
- au moins une étape de régénération de la solution absorbante chargée en composés acides.

L'invention concerne également l'application dudit procédé d'élimination des composés acides au traitement de gaz naturel ou au traitement de gaz d'origine industrielle, notamment au captage du $CO_2$ en post combustion.

**Résumé de l'invention**

L'invention concerne une solution absorbante pour absorber les composés acides d'un effluent gazeux, comprenant :

- de l'eau;
- au moins une amine de formule (I) (nommée N,N,N',N'-tétraméthylhexane-1,6-diamine ou TMHDA)

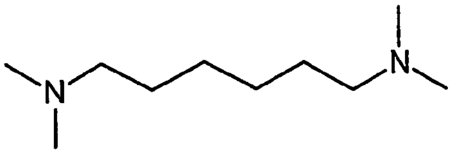

(I)

- au moins une amine primaire ou secondaire de formule (II) ou (III)

Selon l'invention, la formule (II) est de la forme :

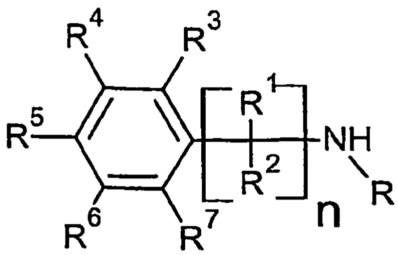

dans laquelle
n=1 ou 2, de préférence n=1
chacun des groupements R1, R2, R3, R4, R5, R6, R7 et R est choisi indépendamment parmi l'un des éléments du groupe constitué de : un atome d'hydrogène, un groupe alkyle de 1 à 2 atomes de carbone. Selon un mode de réalisation de la molécule selon la formule (II), le groupement R est indépendant et donc n'est pas relié à l'un quelconque des groupements R1 à R7. Selon un autre mode de réalisation de la molécule selon la formule (II), le groupement R peut être relié par R3 ou R7 au noyau aromatique de la formule (II), de manière à former un hétérocycle à 5 ou 6 atomes.

Selon l'invention, la formule (III) est de la forme

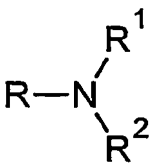

dans laquelle

- R est un groupement alkyle linéaire ou branché de 4 à 8 atomes de carbone,
- les groupements R1 et R2 sont choisis indépendamment parmi l'un des éléments du groupe constitué de :

  - un atome d'hydrogène,
  - un groupe alkyle linéaire ou branché de 1 à 4 atomes de carbone,
  - un groupe

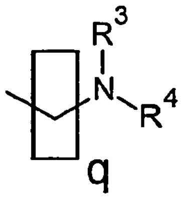

dans laquelle q est égal à 2 ou 3, et les groupements R3, R4 sont choisis indépendamment parmi un atome d'hydrogène ou un groupe alkyle linéaire ou branché de 1 à 4 atomes de carbone.

Selon un mode de réalisation de la molécule selon la formule (III) le groupement R est indépendant et donc le groupement R n'est pas relié au groupement R1 ou R2. Selon un autre mode de réalisation de la molécule selon la formule (III), le groupement R peut être relié à l'un des groupements R1 ou R2 de manière à former un hétérocycle à 5 ou 6 atomes.

Par ailleurs, selon un mode de réalisation de la molécule selon la formule (III), le groupement R3 est indépendant et donc pas relié au groupement R1 ou R2. Selon un autre mode de réalisation de la molécule selon la formule (III), le groupement R3 peut être relié à R1 ou R2 de manière à former un hétérocycle à 5 ou 6 atomes.

La solution absorbante comprend avantageusement de 10 à 90 % poids de N,N,N',N'-tétraméthylhexane-1,6-diamine, préférentiellement de 20 à 60% poids de N,N,N',N'-tétraméthylhexane-1,6-diamine, et de manière très préférée de 30 à 50% poids de N,N,N',N'-tétraméthylhexane-1,6-diamine.

La solution absorbante comprend une quantité non nulle et inférieure à 50% poids, de préférence 20% poids, d'un composé organique activateur de formule (II) ou (III).

De manière très préférée, l'activateur est choisi dans le groupe formé par :

les amines répondant à la formule (II) :

| | |
|---|---|
| 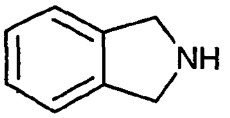 IsoIndoline | 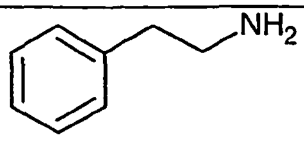 PhénéthylAmine |
| 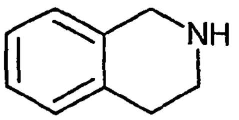 1,2,3,4-TetraHydroIsoquinoline | 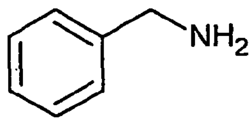 Benzylamine |
| 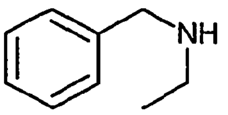 N-EthylBenzylAmine | 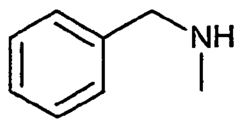 N-MethylBenzylAmine |
| 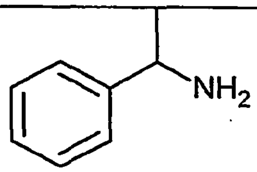 α-MethylBenzylAmine | 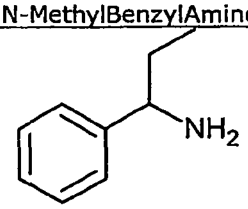 α-EthylBenzylAmine |

et les amines répondant à la formule (III) :

| | |
|---|---|
| 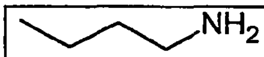 Butylamine | 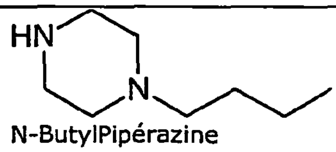 N-ButylPipérazine |

La solution absorbante peut comprendre un solvant physique.

La solution absorbante peut comprendre un acide organique ou inorganique.

L'invention concerne également un procédé d'élimination des composés acides contenus dans un effluent gazeux, dans lequel on effectue :

- une étape d'absorption des composés acides par mise en contact de l'effluent avec une solution absorbante selon l'invention de manière à obtenir un effluent gazeux appauvri en composés acides et une solution absorbante chargée en composés acides,
- au moins une étape de régénération de la solution absorbante chargée en composés acides.

Dans un mode de réalisation du procédé selon l'invention, il est possible d'effectuer l'étape d'absorption de manière à obtenir un effluent gazeux appauvri en composés acides et une solution absorbante chargée en composés acides monophasique, l'étape d'absorption étant suivie d'au moins une étape de séparation liquide-liquide de la solution absorbante chargée en composés acides, diphasique obtenue après chauffage de la solution absorbante, puis d'au moins une étape de régénération fractionnée de la solution absorbante chargée en composés acides.

De manière générale, l'étape d'absorption des composés acides est réalisée à une pression comprise entre 1 bar et 120 bars, et à une température comprise entre 30°C et 100°C.

Compte tenu de la forte stabilité de la (N,N,N',N'-tétraméthylhexane-1,6-diamine), il est possible de régénérer la solution absorbante selon l'invention à haute température dans une colonne à distiller. De manière générale, l'étape de régénération thermique est réalisée à une pression comprise entre 1 bar et 10 bars et une température comprise entre 100°C et 180°C. De manière préférée, la régénération dans la colonne à distiller est réalisée à une température comprise entre 155 et 165°C et à une pression comprise entre 6 et 8,5 bars dans le cas où l'on souhaite réinjecter les gaz acides. De manière préférée, la régénération dans la colonne à distiller est réalisée à une température de 115 et

130°C et à une pression comprise entre 1,7 et 3 bars dans les cas où le gaz acide est envoyé à l'atmosphère ou dans un procédé de traitement aval, comme un procédé Claus ou un procédé de traitement de gaz de queue.

Dans une variante du procédé selon l'invention, on effectue, avant l'étape de régénération, une première étape de détente de la solution absorbante chargée en composés acides.

De manière préférée, on effectue une deuxième étape de détente de la solution absorbante chargée en composés acides, la deuxième étape de détente étant réalisée après la première étape de détente et avant l'étape de régénération, la solution absorbante étant chauffée avant de subir la deuxième étape de détente.

L'invention concerne également un procédé selon l'invention pour le traitement du gaz naturel.

L'invention concerne également un procédé selon l'invention pour le traitement des gaz d'origine Industrielle, de manière préférée pour le captage du $CO_2$.

D'autres caractéristiques et avantages de l'invention seront mieux compris et apparaîtront clairement à la lecture de la description faite, ci-après, en se référant aux figures annexées et données à titre d'exemple:

- la figure 1 représente un schéma de principe d'un procédé de traitement d'effluents de gaz acides.
- la figure 2 représente un schéma de principe d'un procédé de traitement d'effluents de gaz acides avec régénération fractionnée par chauffage.

**Description détaillée de l'invention**

La présente invention propose d'éliminer les composés acides d'un effluent gazeux en mettant en oeuvre la combinaison de deux types de composés amines en solution aqueuse.

La solution absorbante est une solution aqueuse à base de TMHDA, qui a la propriété de réagir de manière réversible avec les composés acides, tels que l'$H_2S$ et le $CO_2$. La TMHDA en phase aqueuse, présente la propriété de former deux phases liquides séparables lorsqu'elle a absorbé une quantité déterminée de composés acides, tels que le $CO_2$. En d'autres termes, la solution aqueuse de TMHDA forme deux phases liquides lorsque son taux de charge (nombre de mole de composés acides captés par une mole d'amine de la solution absorbante) dépasse un taux de charge critique de démixtion, c'est-à-dire un seuil de taux de charge. Lors de la mise en contact dans la colonne d'absorption, le taux de charge de la solution absorbante augmente au fur et à mesure de l'absorption des composés acides contenus dans le gaz. Lors de l'introduction de la solution aqueuse de TMHDA dans la colonne d'absorption, la solution est monophasique. Dans la colonne d'absorption, le taux de charge de la solution absorbante risque de dépasser le taux de charge critique de démixtion et, donc, la solution absorbante risque de se séparer en deux phases. Sous forme de deux phases séparées, le flux de composés acides transférés du gaz vers la solution serait fortement impacté et la hauteur de colonne devrait être adaptée en conséquence. Ce phénomène pose donc de graves problèmes de mise en oeuvre, et compte tenu de la complexité du système, est difficile à modéliser. Pour maintenir la solution absorbante monophasique dans la colonne d'absorption, la présente invention propose de mélanger la TMHDA avec des activateurs spécifiques qui présentent la propriété d'éliminer le phénomène de démixtion par élévation du taux de charge en $CO_2$.

La composition de la solution absorbante selon l'invention est détaillée ci-après.

Les compositions aqueuses à base de N,N,N',N'-TetraMethylHexane-1,6-DiAmine, ou TMHDA, activée par une amine primaire ou secondaire de formule (II) ou (III) présentent un intérêt comme solution absorbante dans l'ensemble des procédés de traitement de gaz acides (gaz naturel, fumées de combustion, etc.).

La molécule N,N,N',N'-tétraméthylhexane-1,6-diamine, présente une capacité d'absorption plus importante avec les gaz acides ($CO_2$, $H_2S$, COS, $SO_2$, $CS_2$ et mercaptans) que les alcanolamines classiquement utilisées. En effet, la N,N,N',N'-Tétraméthylhexane-1,6-diamine présente la particularité d'avoir des taux de charge ($\alpha = n_{gaz\ acide}/n_{amine}$) très importants à de faibles pressions partielles de gaz acide, comparativement aux alcanolamines classiquement utilisées. L'ajout de quelques % poids d'une amine primaire ou secondaire de formule (II) ou (III) à une solution aqueuse de TMHDA, ne modifie que très peu les taux de charge obtenus, tout particulièrement à faible pression partielle de gaz acide. L'utilisation d'une solution absorbante aqueuse selon l'invention permet donc d'économiser sur le coût d'investissement et les coûts de opératoires d'une unité de désacidification (traitement de gaz et captage du $CO_2$).

De plus, la molécule de N,N,N',N'-Tétraméthylhexane-1,6-diamine est intéressante pour sa résistance à la dégradation, notamment thermique. Par conséquent, il est possible de régénérer le solvant à plus forte température et donc d'obtenir un gaz acide à plus forte pression si cela présente un intérêt dans le cas réinjection de gaz acide. Ceci est particulièrement intéressant dans le cas du captage du $CO_2$ en post-combustion où le gaz acide doit être comprimé avant réinjection et séquestration. L'ajout de quelques % poids d'une amine primaire ou secondaire de formule (II) ou (III) ne change pas cette conclusion, car compte tenu de sa faible concentration, la vitesse de dégradation de cette molécule est lente. Par ailleurs, les amines primaires de formule (II) et (III) sont aussi intéressant pour leur résistance à la dégradation. L'utilisation d'une solution absorbante aqueuse selon l'invention permet d'économiser sur les coûts opératoires de l'unité de désacidification, et sur le coût d'investissement et les coûts opératoires associés à la compression du gaz acide.

Par ailleurs, les solutions aqueuses de N,N,N',N'-TetraMethylHexane-1,6-DiAmine, ou TMHDA, activée par une amine primaire ou secondaire de formule (II) ou (III) présentent la particularité de pouvoir être mis en oeuvre dans un procédé de désacidification, avec régénération fractionnée par chauffage tel que décrit dans le document FR 2 898 284.

Synthèse de la N,N,N',N'-TetraMethylHexane-1,6-DiAmine

La N,N,N',N'-TetraMethylHexane-1,6-DiAmine peut être préparée selon différentes voies de synthèse connues de l'homme du métier, décrite par exemple dans les documents JP 1998-341556, EP 1998-105636, JP 1994-286224, JP 1993-25241, EP 1993-118476, EP 1988-309343, JP 1986-124298, JP 1985-147734, DE 1985-3523074, JP 1983-238221, et JP 1983-234589.

Les réactions décrites dans ces documents sont généralement catalytiques, avec des compositions de catalyseurs variées, par exemple : Pt, Pd, Rh, Ru, Cu, Ni, Co. On représente ci-dessous certaines de ces voies identifiées à partir de produits chimiques de base, en notant de manière générique [Cat.] pour l'utilisation d'un catalyseur.

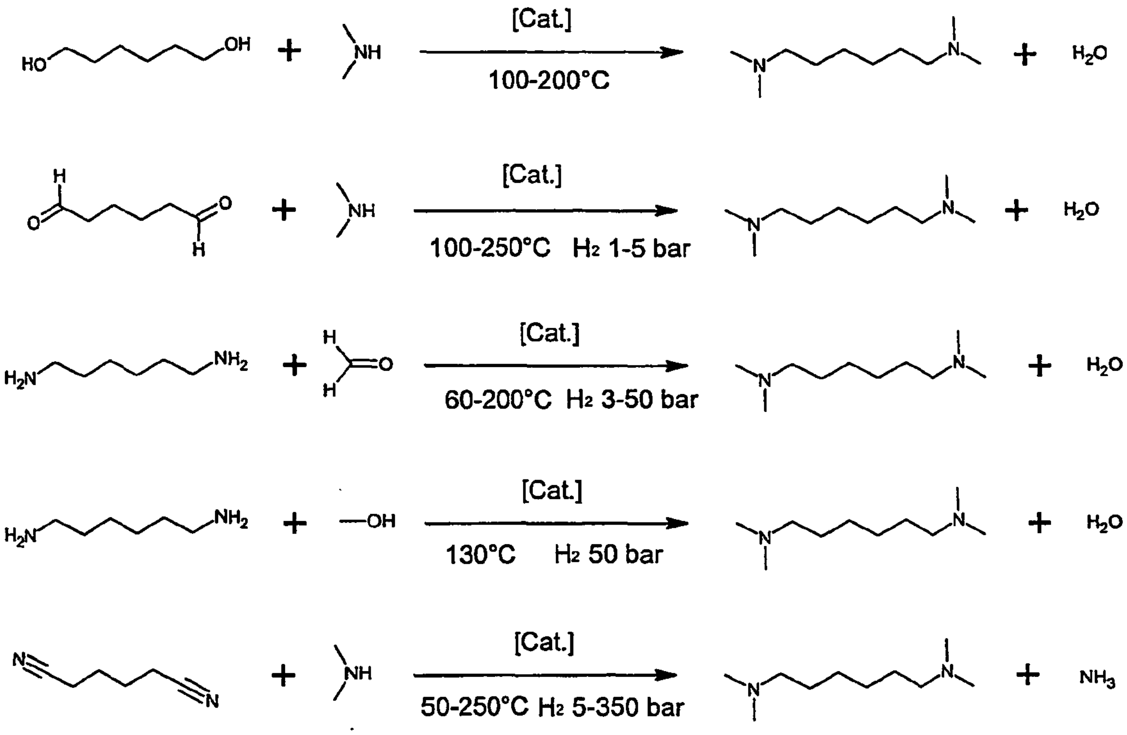


Nature des effluents gazeux

Les solutions absorbantes selon l'invention peuvent être mises en oeuvre pour désacidifier les effluents gazeux suivants : le gaz naturel, les gaz de synthèse, les fumées de combustion, les gaz de raffinerie, les gaz obtenus en queue du procédé Claus, les gaz de fermentation de biomasse, les gaz de cimenterie, les fumées d'incinérateur. Ces effluents gazeux contiennent un ou plusieurs des composés acides suivants : le $CO_2$, l'$H_2S$, des mercaptans, du COS, du $CS_2$.

Les fumées de combustion sont produites notamment par la combustion d'hydrocarbures, de biogaz, de charbon dans une chaudière ou pour une turbine à gaz de combustion, par exemple dans le but de produire de l'électricité. Ces fumées ont une température comprise entre 20 et 60°C, une pression comprise entre 1 et 5 bars et peuvent comporter entre 50 et 80 % d'azote, entre 5 et 40 % de dioxyde de carbone, entre 1 et 20 % d'oxygène, et quelques impuretés comme des SOx et des NOx, s'ils n'ont pas été éliminés en aval du procédé de désacidification.

Le gaz naturel est constitué majoritairement d'hydrocarbures gazeux, mais peut contenir plusieurs des composés acides suivants : le $CO_2$, l'$H_2S$, des mercaptans, du COS, du $CS_2$. La teneur de ces composés acides est très variable et peut aller jusqu'à 40% pour le $CO_2$ et l'$H_2S$. La température du gaz naturel peut être comprise entre 20°C et 100°C. La pression du gaz naturel à traiter peut être comprise entre 10 et 120 bars.

Composition de la solution aqueuse absorbante

La N,N,N',N'-Tétraméthyfhexane-1,6-diamine peut être en concentration variable par exemple comprise entre

10% et 90% poids, de préférence entre 20% et 60% poids, de manière très préférée entre 30% et 50% poids, dans la solution aqueuse.

Les composés de formule générale (II) ou (III) ont une concentration non nulle, par exemple inférieure à 50% poids, voire 30% poids, de préférence inférieure à 20% poids, de manière très préférée inférieure à 10% poids, dans la solution aqueuse.

Une liste non exhaustive des composés de formule générale (II) est donnée ci-dessous :

- BenzylAmine,
- N-MethylBenzylAmine,
- N-EthylBenzylAmine,
- a-MethylBenzylAmine,
- α-EthylBenzylAmine,
- PhénéthylAmine,
- TetraHydroIsoQuinoline
- IsoIndoline.

Une liste non exhaustive des composés de formule générale (III) est donnée ci-dessous :

- ButylAmine,
- N-ButylPipérazine.

La solution absorbante peut contenir au moins 10% poids d'eau, en général entre 10% et 90% poids d'eau, de manière très préférée au moins 50% poids, par exemple entre 60 à 70% poids d'eau.

Dans un mode très préféré, la solution absorbante selon l'invention contient de 62 à 68 % poids d'eau, de 32 à 38% poids d'amines comportant de la N,N,N',N'-Tétraméthylhexane-1,6-diamine en mélange avec au moins une amine primaire ou secondaire de formule (II) ou (III) comme activateur, l'activateur représentant entre 1 et 10% poids de la solution absorbante finale.

Ce type de formulation est particulièrement intéressant dans le cas du captage du $CO_2$ dans les fumées industrielles, ou le traitement du gaz naturel contenant du $CO_2$ au dessus de la spécification désirée. En effet, pour ce type d'applications, on cherche à augmenter la cinétique de captage du $CO_2$, afin de réduire la hauteur des colonnes d'absorption.

Dans un mode de réalisation, la solution absorbante à base de N,N,N',N'-tetramethylhexane-1,6-diamine activée par une amine primaire ou secondaire de formule (II) ou (III) peut comprendre d'autres composés organiques. Ainsi, la solution absorbante selon l'invention peut contenir des composés organiques non réactifs vis à vis des composés acides (couramment nommé "solvants physiques"), qui permettent d'augmenter la solubilité d'au moins un ou plusieurs composés acides de l'effluent gazeux. Par exemple, la solution absorbante peut comporter entre 5% et 50% poids de solvant physique tel que des alcools, des ethers de glycol, des lactames, des pyrrolidones N-alkylées, des pipéridones N-alkylées, des cyclotétraméthylènesulfone, des N-alkylformamides, des N-alkylacétamides, des ethers-cétones ou des phosphates d'alkyles et leur dérivés. A titre d'exemple et de façon non limitative, il peut s'agir du méthanol, du tetraethylèneglycoldimethylether, du sulfolane ou de la N-formyl morpholine.

Dans un mode de réalisation, la solution absorbante à base de N,N,N',N'-tetramethylhexane-1,6-diamine activée par une amine primaire ou secondaire de formule (II) ou (III) peut comprendre un acide organique ou inorganique. Une liste non exhaustive de composés acides pouvant être utilisés à cet effet est donnée ci-dessous :

- acide formique
- acide oxalique
- acide acétique
- acide propanoïque
- acide butanoïque
- acide aminé (glycine, taurine, etc.)
- acide phosphorique
- acide phosphoreux
- acide pyrophosphorique
- acide sulfurique
- acide sulfureux
- acide nitreux
- acide chlorhydrique.

Procédé d'élimination des composés acides dans un effluent gazeux (Fig. 1)

La mise en oeuvre d'une solution absorbante pour désacidifier un effluent gazeux est réalisée de façon schématique en effectuant une étape d'absorption suivie d'une étape de régénération. L'étape d'absorption consiste à mettre en contact l'effluent gazeux contenant les composés acides à éliminer avec la solution absorbante dans une colonne d'absorption C1. L'effluent gazeux à traiter (~1) et la solution absorbante (~4) alimentent la colonne C1. Lors du contact, les composés organiques munis d'une fonction amine de la solution absorbante (~4) réagissent avec les composés acides contenus dans l'effluent (~1) de manière à obtenir un effluent gazeux appauvri en composés acides (~2) qui sort en tête de colonne C1 et une solution absorbante enrichie en composés acides (~3) qui sort en fond de colonne C1. La solution absorbante enrichie en composés acides (~3) est envoyée vers un échangeur E1, où elle est réchauffée par le flux (~6) provenant de la colonne de régénération C2. La solution absorbante chargée et réchauffée en sortie de l'échangeur E1 (~5) alimente la colonne à distiller (ou colonne de régénération) C2 dans laquelle a lieu la régénération de la solution absorbante chargée en composés acides. L'étape de régénération consiste donc notamment à chauffer et, éventuellement à détendre, la solution absorbante enrichie en composés acides afin de libérer les composés acides qui sortent en tête de colonne C2 sous forme gazeuse (~7). La solution absorbante régénérée, c'est-à-dire appauvrie en composés acides (~6), sort en fond de colonne C2, puis passe dans l'échangeur E1, dans lequel elle cède de la chaleur au flux (~3) comme décrit précédemment. La solution absorbante régénérée et refroidie (~4) est ensuite recyclée vers la colonne d'absorption C1.

L'étape d'absorption des composés acides peut être réalisée à une pression comprise entre 1 bar et 120 bars, de préférence entre 20 bars et 100 bars pour le traitement d'un gaz naturel, de préférence entre 1 et 3 bars pour le traitement des fumées industrielles, et à une température comprise entre 20°C et 100°C, préférentiellement comprise entre 30°C et 90°C, de manière encore plus préférée entre 30°C et 60°C. En effet, le procédé selon l'invention présente une excellente capacité d'absorption des composés acides lorsque la température dans la colonne d'absorption C1 est comprise entre 30°C et 60°C

L'étape de régénération du procédé selon l'invention peut être réalisée par régénération thermique, éventuellement complétée par une ou plusieurs étapes de détente.

Compte tenu de la forte stabilité de la N,N,N',N'-tétraméthylhexane-1,6-diamine, il est possible de régénérer la solution absorbante selon l'invention à haute température dans une colonne à distiller. De manière générale, l'étape de régénération thermique est réalisée à une température comprise entre 100°C et 180°C, de préférence comprise entre 130 °C et 170 °C, et à une pression comprise entre 1 bar et 10 bars. De manière préférée, la régénération dans la colonne à distiller est réalisée à une température comprise entre 155 et 165°C et à une pression comprise entre 6 et 8,5 bars dans le cas où l'on souhaite réinjecter les gaz acides. De manière préférée, la régénération dans la colonne à distiller est réalisée à une température de 115 et 130°C et à une pression comprise entre 1,7 et 3 bars dans les cas où le gaz acide est envoyé à l'atmosphère ou dans un procédé de traitement aval, comme un procédé Claus ou un procédé de traitement de gaz de queue.

Par ailleurs, un phénomène de démixtion pour une solution absorbante donnée (séparation de phases liquide-liquide au sein de la solution absorbante) peut être induit par une élévation de la température. Ledit phénomène de démixtion peut être contrôlé par le choix des conditions opératoires du procédé et/ou de la composition de la solution absorbante. Dans ce cas, des variantes (Fig. 2) du procédé selon l'invention peuvent être mises en oeuvre, notamment une régénération fractionnée par chauffage de la solution absorbante.

Procédé d'élimination des composés acides dans un effluent gazeux avec régénération fractionnée par chauffage (Fig. 2)

La mise en oeuvre d'une solution absorbante pour désacidifier un effluent gazeux est réalisée de façon schématique en effectuant une étape d'absorption, suivie d'une étape de chauffage de la solution absorbante, suivie d'une étape de séparation liquide-liquide de la solution absorbante, suivie d'une étape de régénération. L'étape d'absorption consiste à mettre en contact l'effluent gazeux contenant les composés acides à éliminer avec la solution absorbante dans une colonne d'absorption C1. L'effluent gazeux à traiter (~1) et la solution absorbante (~4) alimentent la colonne C1. Lors du contact, les composés organiques munis d'une fonction amine de la solution absorbante (~4) réagissent avec les composés acides contenus dans l'effluent (~1) de manière à obtenir un effluent gazeux appauvri en composés acides (~2) qui sort en tête de colonne C1 et une solution absorbante enrichie en composés acides (~3) qui sort en fond de colonne C1. L'étape de chauffage consiste à élever la température de la solution absorbante (~3), en passant par exemple dans un échangeur thermique E1, pour obtenir une solution diphasique (~5). La solution diphasique (~5) est envoyée vers un décanteur BS1, dans lequel s'effectue l'étape de séparation liquide-liquide qui consiste à séparer les deux phases obtenues dans l'étape de chauffage en envoyant la phase riche en gaz acide (~12) vers la colonne de régénération C2, et renvoyant la phase pauvre en gaz acide (~14), après passage éventuel dans un échangeur E3, vers la colonne d'absorption C1.

La phase gazeuse libérée par chauffage de la solution absorbante (~3) dans l'échangeur E1 est séparée des

phases liquides dans BS1 et évacué par le conduite (-13).

L'étape de régénération consiste donc notamment à chauffer dans la colonne de distillation C2 et, éventuellement à détendre, la solution absorbante enrichie en composés acides (~12) afin de libérer les composés acides qui sortent en tête de colonne C2 sous forme gazeuse (~7). La solution absorbante régénérée, c'est-à-dire appauvrie en composés acides (~6), sort en fond de colonne C2, puis passe dans l'échangeur E1, dans lequel elle cède de la chaleur au flux (~3) comme décrit précédemment. La solution absorbante régénérée et refroidie (~4), après passage éventuel dans un nouvel échangeur E2, est ensuite recyclée vers la colonne d'absorption C1. En fond de la colonne de distillation C2, une portion de la solution absorbante est prélevée par le conduit (~10), chauffée dans le rebouilleur R1, puis réintroduite dans le fond de la colonne C2 par le conduit (-11).

L'étape d'absorption des composés acides peut être réalisée à une pression comprise entre 1 bar et 120 bars, de préférence entre 20 bars et 100 bars pour le traitement d'un gaz naturel, de préférence entre 1 et 3 bars pour le traitement des fumées industrielles, et à une température comprise entre 20°C et 100°C, préférentiellement comprise entre 30°C et 90°C, de manière encore plus préférée entre 30°C et 60°C. En effet, le procédé selon l'invention présente une excellente capacité d'absorption des composés acides lorsque la température dans la colonne d'absorption C1 est comprise entre 30°C et 60°C

L'étape de régénération du procédé selon l'invention peut être réalisée par régénération thermique, éventuellement complétée par une ou plusieurs étapes de détente.

Compte tenu de la forte stabilité de la N,N,N',N'-tétraméthylhexane-1,6-diamine, il est possible de régénérer la solution absorbante selon l'invention à haute température dans une colonne à distiller. De manière générale, l'étape de régénération thermique est réalisée à une température comprise entre 100°C et 180°C et à une pression comprise entre 1 bar et 10 bars. De manière préférée, la régénération dans la colonne à distiller est réalisée à une température comprise entre 155 et 165°C et à une pression comprise entre 6 et 8,5 bars dans le cas où l'on souhaite réinjecter les gaz acides. De manière préférée, la régénération dans la colonne à distiller est réalisée à une température de 115 et 130°C et à une pression comprise entre 1,7 et 3 bars dans les cas où le gaz acide est envoyé à l'atmosphère ou dans un procédé de traitement aval, comme un procédé Claus ou un procédé de traitement de gaz de queue.

**Exemples :**

On utilise comme solutions absorbantes dans ces exemples des solutions aqueuses de N,N,N',N'-Tétraméthylhexane-1,6-diamine en association avec un composé de formule générale (II) ou (III). Les composés de formule générale (II) utilisés à titre d'exemple sont la TetraHydroIsoQuinoline (THIQ) et la N-MethylBenzylAmine (N-MetBzA). Les composés de formule générale (III) utilisés à titre d'exemple sont la N-ButylPipérazine (N-ButPz) et la n-butylamine.

| | |
|---|---|
| Formulation A | TMHDA + THIQ + $H_2O$ |
| Formulation B | TMHDA + N-MetBzA + $H_2O$ |
| Formulation C | TMHDA + N-ButPz + $H_2O$ |
| Formulation D | TMHDA + n-butylamine + $H_2O$ |

Dans un premier temps, on montrera que les propriétés physico-chimiques des formulations A, B, C et D (i.e. équilibre liquide-liquide) sont très différentes de celles de solutions aqueuses de N,N,N',N'-Tétraméthylhexane-1,6-diamine ou de celles de solutions aqueuses de N,N,N',N'-Tétraméthylhexane-1,6-diamine en association avec une amine primaire ou secondaire ne répondant pas à la formule générale (II) ou (III). Ces composés choisis à titre d'illustration sont le Pipérazine (Pz), la 3-MethylAminoPropaneAmine (MAPA), la DiEthanolAmine (DEA) et la MonoEthanolAmine (MEA).

| | |
|---|---|
| Formulation E | TMHDA + $H_2O$ |
| Formulation F | TMHDA + Pz + $H_2O$ |
| Formulation G | TMHDA + MAPA + $H_2O$ |
| Formulation H | TMHDA + DEA + $H_2O$ |
| Formulation I | TMHDA + MEA + $H_2O$ |

Dans un deuxième temps, on montrera que les performances des formulations A, B et C (i.e. capacité de captage, stabilité) sont sensiblement équivalentes à celle d'une solution aqueuse de TMHDA à 35%poids (soit la formulation E).

Leurs performances seront alors comparées à celle d'une solution aqueuse de MonoEthanolAmine à 30% poids, qui constitue le solvant de référence pour une application de captage des fumées en post-combustion, et à celle d'une solution aqueuse de MéthylDiEthanolAmine à 40% poids qui constitue le solvant de référence pour une application de traitement du gaz naturel.

Enfin, leurs performances (i.e. capacité de captage, stabilité) seront comparées à celle d'une solution aqueuse de N,N,N',N'-Tétraméthylpropane-1,3-diamine (TMPDA) à 35%, une autre diamine tertiaire de structure ressemblante à la celle de la N,N,N',N'-Tétraméthylhexane-1,6-diamine, pour une application de captage des fumées en post-combustion.

Exemple 1 : Equilibre liquide-liquide

Le phénomène de démixtion peut être contrôlé par la nature de l'activateur qui est ajouté à une solution aqueuse de N,N,N',N'-Tétraméthylhexane-1,6-diamine.

Suivant la composition de la solution absorbante à base de N,N,N',N'-Tétraméthylhexane-1,6-diamine et la présence éventuelle d'une amine primaire ou secondaire, la composition du gaz à traiter (i.e. la pression partielle de $CO_2$) et la température de la solution absorbante, une séparation de phase liquide-liquide peut se produire (phénomène de démixtion). On détermine par des tests au laboratoire (dans des réacteurs parfaitement agités gaz-liquide) les conditions dans lesquelles se produit la démixtion pour une formulation donnée (i.e. concentrations des amines et de l'eau donnée), à une température donnée, en augmentant progressivement la pression partielle de $CO_2$ et donc le taux de charge en $CO_2$ ($\alpha = n_{gaz\ acide}/n_{amine}$) à l'équilibre. Par ailleurs, lorsque le solvant est diphasique, les deux phases liquides peuvent être prélevées et analysées pour déterminer leur composition (analyse chromatographique, dosage acido-basique ou volumétrique).

Selon les résultats de ces tests de laboratoire, la solution absorbante peut être mise en oeuvre dans un procédé de désacidification, ou dans un procédé de désacidification avec régénération fractionnée par chauffage, tel que décrit sur la figure 2. Une solution absorbante à base de N,N,N',N'-Tétraméthylhexane-1,6-diamine activée par un composé de formule générale (II) ou (III) est particulièrement adaptée à ce type de procédé, car elle permet d'être monophasique dans les conditions opératoires correspondant à celle de l'absorbeur (i.e. généralement 40°C), et diphasique au delà de l'échangeur charge/effluent (i.e. généralement 90°C), et avec une séparation de phase liquide-liquide rapide.

1/ Equilibre liquide-liquide à 40°C

On s'intéresse ici aux équilibres liquide-liquide à 40°C, ce qui correspond aux températures basses d'un absorbeur. Trois exemples permettent de mettre en évidence l'importance de la structure de l'activateur, de la concentration respective de l'activateur et de la TMHDA, et de la concentration totale d'amine.

1-A/ Structure de l'activateur

On s'intéresse ici aux équilibres liquide-liquide à 40°C, ce qui correspond aux températures basses d'un absorbeur. Les tests au laboratoire sont réalisés pour des formulations ayant une concentration totale de 35%pds. d'amine. Le tableau ci-dessous résume les résultats obtenus pour différentes formulations.

| Formulation | [TMHDA] (%pds.) | [Activateur] (%pds.) | Taux de charge où l'on rentre dans la zone diphasique | Taux de charge où l'on sort de la zone diphasique |
|---|---|---|---|---|
| Formulation A | 30 | 5 | pas de démixtion par élévation du taux de charge pour une $PPCO_2$ < 0,5 bar | |
| Formulation B | 30 | 5 | pas de démixtion par élévation du taux de charge pour une $PPCO_2$ < 0,5 bar | |
| Formulation C | 30 | 5 | pas de démixtion par élévation du taux de charge pour une $PPCO_2$ < 0,5 bar | |
| Formulation E | 35 | 0 | 0,54 | 1,50 |
| Formulation F | 30 | 5 | 0,64 | 0,78 |
| Formulation G | 30 | 5 | 0,44 | 0,87 |

On peut remarquer à l'aide du tableau ci-dessous qu'une quantité égale à 5% des activateurs de formule générale (II) ou (III) permet d'éliminer totalement le phénomène de démixtion (Formulations A, B et C) qui est observée en absence d'activateur (Formulation E). Les formulations A, B et C ci-dessus sont donc intéressantes, car elles permettent d'avoir une solution absorbante monophasique dans les conditions opératoires correspondant à l'absorbeur.

En revanche, on voit que les activateurs ne répondant pas à la formule générale (II) ou (III) ne permettent pas d'éliminer totalement le phénomène de démixtion (Formulations F et G).

1-B/ Concentration de l'activateur

On s'intéresse ici aux équilibres liquide-liquide à 40°C, ce qui correspond aux températures basses d'un absorbeur. Les tests au laboratoire sont réalisés pour des formulations de type A ayant une concentration totale de 35%pds. d'amine. Le tableau ci-dessous résume les résultats obtenus pour différentes concentrations de TetraHydroIsoQuinoline.

| [TMHDA (%pds.) | [THIQ] (%pds.) | Taux de charge où l'on rentre dans la zone diphasique | Taux de charge où l'on sort de la zone diphasique |
|---|---|---|---|
| 35,0 | 0,0 | 0,54 | 1,50 |
| 34,0 | 1,0 | 0,77 | 1,45 |
| 32,5 | 2,5 | pas de démixtion par élévation du taux de charge pour une $PPCO_2 < 0,5$ bar | |
| 30,0 | 5,0 | pas de démixtion par élévation du taux de charge pour une $PPCO_2 < 0,5$ bar | |

L'effet observé avec les activateurs de formule générale (II) ou (III) est d'autant plus surprenant, qu'une très faible quantité d'activateur (i.e. moins de 2,5%pds) comme la TetraHydroIsoQuinoline (Formulation A) permet d'éliminer totalement le phénomène de démixtion qui est observé en absence d'activateur (Formulation E).

1-C/ Concentration totale d'amine

On s'intéresse ici aux équilibres liquide-liquide à 40°C, ce qui correspond aux températures basses d'un absorbeur. Les tests au laboratoire sont réalisés pour des formulations ayant une concentration totale de 56%pds. d'amine. Le tableau ci-dessous résume les résultats obtenus pour différentes formulations.

On peut tout d'abord remarquer que le phénomène de démixtion qui est observé en absence d'activateur (Formulation E), est d'autant plus marqué que la concentration totale d'amine est importante. Pour une concentration totale de 35%pds. (cas décrit dans l'exemple 1-A/), on entre dans la zone diphasique pour un taux de charge de 0,54 et on en sort pour un taux de charge de 1,50. Pour une concentration totale de 56%pds. (cas d'écrit dans l'exemple 1-C/), on entre dans la zone diphasique pour un taux de charge de 0,1 et il est difficile de déterminer le taux de charge pour lequel on sort de la zone diphasique avec les équipements dont nous disposons.

| Formulation | [TMHDA] (%pds.) | [Activateur] (%pds.) | Taux de charge où l'on rentre dans la zone diphasique |
|---|---|---|---|
| Formulation B | 50 | 6 | pas de démixtion par élévation du taux de charge |
| Formulation D | 50 | 6 | pas de démixtion par élévation du taux de charge |
| Formulation E | 56 | 0 | 0,10 |
| Formulation H | 50 | 6 | 0,12 |
| Formulation I | 50 | 6 | 0,23 |

On peut remarquer par ailleurs à l'aide du tableau ci-dessous que 6% des activateurs de formule générale (II) ou (III) permettent d'éliminer totalement le phénomène de démixtion (Formulations B et D) qui est observé en absence d'activateur (Formulation E). Les formulations B et D ci-dessus sont donc intéressantes car elles permettent d'avoir une solution absorbante monophasique dans les conditions opératoires correspondant à l'absorbeur.

En revanche, on voit que les activateurs ne répondant pas à la formule générale (II) ou (III) ne permettent pas d'éliminer totalement le phénomène de démixtion (Formulations H et I).

2/ Equilibre liquide-liquide à 90°C

On s'intéresse maintenant aux équilibres liquide-liquide à 90°C, qui correspond aux températures typiques après passage dans l'échangeur charge/effluent dans un procédé de désacidification.

Suivant la composition de la solution absorbante à base de N,N,N',N'-Tétraméthylhexane-1,6-diamine et la présence éventuelle d'une amine primaire ou secondaire, la composition du gaz à traiter et la température de la solution absorbante, une séparation de phase liquide-liquide peut se produire (phénomène de démixtion).

A titre d'illustration, on donne ci-dessous la composition (i.e. concentration des amines et taux de charge = nombre de moles de gaz acide captées rapporté au nombre de moles d'amine) des phases inférieures et supérieures obtenues pour les formulations de type A, par exemple pour une concentration de N,N,N',N'-Tétraméthylhexane-1,6-diamine de 30%pds. et une concentration de TetraHydroIsoQuinoline de 5%pds., à 90°C, pour différentes pressions partielles de gaz acide en équilibre avec la solution absorbante.

| PP CO2 (bar) | Phase | [TMHDA] (%pds.) | [THIQ] (%pds.) | [Amine] (%pds.) | Taux de charge |
|---|---|---|---|---|---|
| 0 | inférieure | 1,9 | 0,4 | 2,3 | 0 |
| | supérieure | 69,9 | 9,1 | 79,0 | 0 |
| 0,1 | inférieure | 9,5 | 2,2 | 11,7 | 0,62 |
| | supérieure | 69,9 | 9,1 | 79,0 | 0,03 |
| 0,3 | inférieure | 16,9 | 3,5 | 20,4 | 0,67 |
| | supérieure | 73,6 | 6,5 | 80,1 | 0,06 |
| 1 | inférieure | 19,8 | 3,7 | 23,5 | 0,78 |
| | supérieure | 73,8 | 5,1 | 78,9 | 0,09 |

On peut tout d'abord remarquer que les phases supérieures qui sont obtenues pour différentes pressions partielles de $CO_2$, ont une concentration d'amine très élevées, et des taux de charge très faible (inférieure à 0,1). A l'inverse, les phases inférieures qui sont obtenues pour différentes pressions partielles de $CO_2$, ont une concentration d'amine faible à modérée, et des taux de charge élevés. Plus la pression partielle de $CO_2$ à l'équilibre est élevée, plus la concentration d'amine dans la phase inférieure est élevée.

Les phases qui sont obtenues après passage dans l'échangeur charge / effluent peuvent donc être séparées, et la phase supérieure qui est pauvre en $CO_2$ (i.e. phase supérieure) peut-être recyclée directement vers l'absorbeur, alors que la phase riche en $CO_2$ (i.e. phase inférieure) doit être envoyée vers une étape de régénération. Cette opération permet de réduire le débit de solvant dans le régénérateur, et donc réduire l'énergie de régénération du solvant.

Par ailleurs, on peut noter que la concentration de l'activateur est toujours plus élevée dans la phase supérieure que dans la phase inférieure. Ceci constitue un autre avantage des formulations de type A, puisque que l'on peut augmenter davantage la concentration de l'activateur pour augmenter la cinétique de captage du $CO_2$, sans que ce dernier pénalise l'étape de régénération. En effet, de part la séparation liquide-liquide réalisée après l'échangeur charge / effluent, une partie de l'activateur circule en boucle dans l'absorbeur.

Exemple 2 : Capacité de captage

1/ Impact de l'activateur sur les capacités de captage

On s'intéresse ici à la capacité de captage des solutions aqueuses de TMHDA activées par une amine primaire ou secondaire de formule générale (II) ou (III). A titre d'exemple, le tableau ci-dessous permet de comparer les taux de charge ($\alpha = n_{gaz\ acide}/n_{amine}$) obtenus à 40°C pour différentes pressions partielles de $CO_2$ pour une concentration totale d'amine de 35%, sans (Formulation E) et avec différents activateurs de formule générale (II) ou (III) (Formulations A, B et C).

| Formulation | [TMHDA] (%pds.) | [Activateur] (%pds.) | T (°C) | Taux de charge α = $n_{CO2}/n_{amine}$ | $PP_{CO2}$ = 0,1 bar | $PP_{CO2}$ = 0,3 bar | $PP_{CO2}$ = 1 bar |
|---|---|---|---|---|---|---|---|
| Formulation E | 35 | 0 | 40 | | 1,18 | 1,68 | 1,88 |
| Formulation A | 30 | 5 | 40 | | 1,12 | 1,44 | 1,55 |
| Formulation B | 30 | 5 | 40 | | 1,29 | 1,52 | 1,67 |
| Formulation C | 30 | 5 | 40 | | 1,25 | 1,55 | 1,71 |

On peut remarquer que la capacité de captage de la TMHDA est très peu affectée par la substitution de quelques pourcents poids de la TMHDA par quelques pourcents poids d'activateur, notamment à faible pression partielle de $CO_2$.

Par souci de simplification, on comparera dans le paragraphe suivant uniquement les propriétés d'une solution aqueuse de TMHDA à 35%pds (Formulation E) à celle des amines généralement utilisées pour les applications de traitement du gaz naturel et de captage du $CO_2$.

2/ Comparaison des capacités de captage avec d'autres solutions absorbantes

A titre d'exemple, on peut comparer les taux de charge ($\alpha = n_{gaz\ acide}/n_{amine}$) obtenus à 40°C pour différentes pressions partielles de $CO_2$ entre une solution aqueuse de TMHDA à 35%pds (Formulation E) et une solution absorbante de MonoEthanolAmine à 30% en poids et de TMPDA à 35% en poids :

| Amine | Concentration | T (°C) | Taux de charge α = $n_{CO2}/n_{amine}$ | $PP_{CO2}$ = 0,1 bar | $PP_{CO2}$ = 0,3 bar | $PP_{CO2}$ = 1 bar |
|---|---|---|---|---|---|---|
| TMHDA | 35 % poids | 40 | | 1,18 | 1,68 | 1,88 |
| TMPDA | 35 % poids | 40 | | 0,86 | 1,13 | 1,51 |
| MEA | 30 % poids | 40 | | 0,52 | 0,55 | 0,6 |

A titre d'exemple, on peut comparer les taux de charge ($\alpha = n_{gaz\ acide}/n_{amine}$) obtenus à 40°C pour différentes pressions partielles de $H_2S$ entre une solution aqueuse de TMHDA à 35%pds (Formulation E) et une solution absorbante de MéthylDiEthanolAmine à 40% en poids :

| Amine | Concentration | T (°C) | Taux de charge α = $n_{H2S}/n_{amine}$ | $PP_{H2S}$ = 0,01 bar | $PP_{H2S}$ = 0,03 bar | $PP_{H2S}$ = 0,1 bar |
|---|---|---|---|---|---|---|
| TMHDA | 35 % poids | 40 | | 0,55 | 0,98 | 1,76 |
| MDEA | 40 % poids | 40 | | 0,11 | 0,20 | 0,37 |

3/ Conclusion

Ces exemples montrent les taux de charge importants qui peuvent être obtenus grâce des solutions aqueuses de TMHDA activées par une amine primaire ou secondaire de formule générale (II) ou (III), notamment pour des faibles pressions partielles de gaz acides.

Exemple 3 : Stabilité

La molécule de N,N,N',N'-Tétraméthylhexane-1,6-diamine présente la particularité d'être très résistante aux

dégradations qui peuvent se produire dans une unité de désacidification.
Les activateurs de formule générale (II) et (III) présentent eux aussi la particularité d'être très résistants aux dégradations qui peuvent se produire dans une unité de désacidification.

A l'échelle du laboratoire, des solutions aqueuses d'amine peuvent être dégradée au sein de réacteur fermées, chauffés à une température T, et mis sous pression une pression partielle PP de différents gaz ($CO_2$, $O_2$, $H_2S$, $N_2$). La phase liquide est agitée à l'aide d'un barreau aimanté. Au bout d'une certaine durée, un échantillon de la phase liquide peut être prélevé, puis analysé par différentes technique, notamment par chromatographie en phase gazeuse. Le tableau ci-dessous donne le taux de dégradation TD de la solution absorbante, dans différentes conditions, pour une durée de 15 jours, défini par l'équation ci-dessous :

$$TD(\%) = \frac{[Amine] - [Amine]^\circ}{[Amine]^\circ}$$

où [Amine] est la concentration de l'amine dans l'échantillon dégradé, et [Amine]° est la concentration de l'amine dans la solution non dégradée.
Plus le taux de dégradation TD est faible, toutes choses égales par ailleurs, plus on peut considérer que l'amine est stable.

Pour une formulation comprenant de la TMHDA et un activateur de formule générale (II) ou (III), la concentration des activateurs peut être très faible (par exemple 2,5% poids, cf. exemple 1 section 1-B/). Par ailleurs, dans le cas d'une mise en oeuvre du procédé avec régénération fractionnée par chauffage, une grande partie de l'activateur tourne en boucle dans l'absorbeur (cf. exemple 1 section 2/). Ainsi, la concentration d'activateur dans le régénérateur, où la majeure partie des réactions de dégradation ont lieu compte tenu de forte température, peut être extrêmement faible. Ces arguments convergent vers le fait que ces activateurs se dégraderont faiblement dans le procédé d'absorption selon l'invention.

Étant donné que les tests de dégradation sont réalisés dans des conditions très sévères, de nombreux produits de dégradation peuvent être générés. Lorsqu'on réalise un test de dégradation sur une formulation de TMHDA acitvée par quelques % pds d'une amine primaire ou secondaire, il est donc très difficile de quantifier avec certitude le taux de dégradation de l'activateur. Par conséquent, la stabilité de l'activateur seul est testé lors d'une campagne d'essai indépendante.

On présente tout d'abord les résultats obtenus pour la TMHDA seule, puis pour les activateurs de formule générale (II) seuls.

1/ Stabilité de la TMHDA

Le tableau ci-dessous donne le taux de dégradation TD de différentes solutions aqueuses d'amine, pour une température de 140°C, en absence et en présence de différents gaz acides.

| Amine | Concentration | T (°C) | Taux de dégradation | Tension de vapeur du solvant | $PP_{CO2}$ = 20 bar | $PP_{O2}$ = 4,2 bar | $PP_{CO2}$ = 16,6 bar + $PP_{H2S}$ = 3,4 bar |
|---|---|---|---|---|---|---|---|
| TMHDA | 35 % poids | 140 | | 0 % | <3 % | 5 % | <3% |
| TMPDA | 35 % poids | 140 | | 6,7 % | 21 % | 12 % | - |
| MDEA | 40 % poids | 140 | | 0 % | 25 % | 14 % | 29 % |
| MEA | 30 % poids | 140 | | 5,4 % | 42 % | 21 % | - |

Le tableau ci-dessous donne le taux de dégradation TD de différentes solution aqueuses d'amine, pour une température de 180°C, en absence et en présence de gaz acide, qui est représentative des dégradations qui pourraient se produire en fond de régénérateur, si l'on souhaite obtenir un gaz acide à forte pression pour des applications de réinjection.

| Amine | Concentration | T (°C) | Taux de dégradation | Tension de vapeur du solvant | $PP_{CO2}$ = 20 bar |
|---|---|---|---|---|---|
| TMHDA | 35 % poids | 180 | | 5 % | 5 % |
| MDEA | 40 % poids | 180 | | 26 % | 77 % |

Cet exemple montre que l'utilisation d'une solution absorbante composée majoritairement de la N,N,N',N'-Tétraméthylhexane-1,6-diamine, permet d'obtenir un faible taux de dégradation par rapport aux solutions absorbantes à base d'amines de l'art antérieur (MéthylDiEthanolAmine et MonoEthanolAmine). On peut par ailleurs remarquer qu'elle est beaucoup plus stable qu'une molécule de structure très proche, comme la N,N,N',N'-TetraMéthylPropane-1,3-diamine (TMPDA).

2/ Stabilité des activateurs de formule générale (II)

Le tableau ci-dessous donne le taux de dégradation TD de différentes solutions aqueuses d'activateurs comme la TetraHydroIsoQuinoline et la N-MethylBenzylamine répondant à la formule générale (II), et de différentes solutions aqueuses d'activateurs bien connus de l'homme du métier, pour une température de 140°C d'une part en présence de $CO_2$ et d'autre part en présence d'$O_2$.

| Amine | Concentration | T (°C) | Taux de dégradation | $PP_{CO2}$ = 20 bar | $PP_{O2}$ = 4,2 bar |
|---|---|---|---|---|---|
| THIQ | 4M | 140 | | 5% | 10% |
| N-MBzA | 4M | 140 | | 11% | 9% |
| DEA | 4M | 140 | | 93% | 22% |
| MEA | 4M | 140 | | 42% | 21% |

Cet exemple montre que l'utilisation d'amines primaires ou secondaires répondant à la formule générale (II) permet d'obtenir un faible taux de dégradation par rapport activateurs de l'art antérieur (DiEthanolAmine et MonoEthanolAmine), en présence de $CO_2$, mais aussi en présence d'oxygène contenu par exemple dans les fumées de combustion.

3/ Conclusion

Par conséquent, il est possible de régénérer la solution absorbante selon l'invention à plus forte température et donc d'obtenir un gaz acide à plus forte pression. Ceci est particulièrement intéressant dans le cas du captage du $CO_2$ en post-combustion où le gaz acide doit être comprimé pour être liquéfié avant réinjection.

**Revendications**

**1.** Procédé d'élimination des composés acides contenus dans un effluent gazeux, dans lequel :

- on effectue une étape d'absorption des composés acides par mise en contact de l'effluent avec une solution absorbante de manière à obtenir un effluent gazeux appauvri en composés acides et une solution absorbante chargée en composés acides, la solution absorbante comprenant :

a. de l'eau
b. au moins une amine de formule (I) (N,N,N',N'-tétraméthylhexane-1,6-diamine)

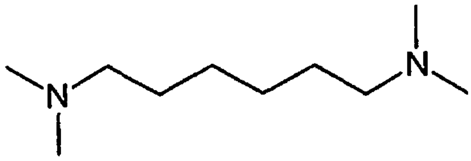

(I)

c. au moins un composé activateur choisi parmi la N-ButylPipérazine et un composé répondant à la formule (II)

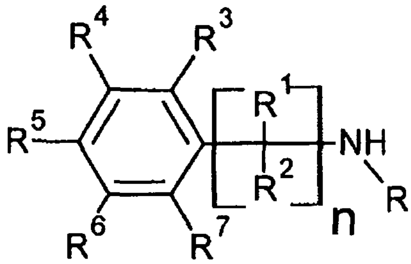

dans laquelle n=1 ou 2, et
dans laquelle chacun des groupements R1, R2, R3, R4, R5, R6, R7 et R est choisi indépendamment parmi l'un des éléments du groupe constitué de : un atome d'hydrogène, un groupe alkyle de 1 à 2 atomes de carbone ,
et dans laquelle, le groupement R possède l'une des caractéristiques suivantes :

- le groupement R n'est pas relié à l'un quelconque des groupements R1 à R7,
- le groupement R est relié par R3 ou par R7 au noyau aromatique de la formule (II) de manière à former un hétérocycle à 5 ou 6 atomes, et
- on effectue une étape de régénération dans laquelle on envoie au moins une partie de la solution chargée en composés acides dans une colonne à distiller pour libérer les composés acides sous forme d'un effluent gazeux et obtenir une solution absorbante régénérée

**2.** Procédé selon la revendication 1, dans lequel la solution absorbante comporte de 10% à 90 % poids de N,N,N',N'-tétraméthylhexane-1,6-diamine.

**3.** Procédé selon l'une des revendications 1 et 2, dans lequel la solution absorbante comporte une quantité non nulle et inférieure à 50 % poids du ou des composés activateurs.

**4.** Procédé selon l'une des revendications 1 à 3, dans lequel le ou les composés activateurs sont choisis dans le groupe formé par :

- BenzylAmine,
- N-MethylBenzylAmine,
- N-EthylBenzylAmine,
- α-MethylBenzylAmine,
- α-EthylBenzylAmine,
- PhénétylAmine
- TetraHydroIsoQuinoline,
- IsoIndoline,
- N-ButylPipérazine.

**5.** Procédé selon l'une des revendications 1 à 4, dans lequel la solution absorbante comporte un solvant physique.

**6.** Procédé selon l'une des revendications 1 à 5, dans lequel la solution absorbante comporte un acide organique ou inorganique.

**7.** Procédé selon l'une des revendications précédentes, dans lequel l'étape d'absorption des composés acides est

réalisée à une pression comprise entre 1 bar et 120 bars, et à une température comprise entre 30°C et 100°C.

8. Procédé selon l'une des revendications précédentes, dans lequel l'étape d'absorption des composés acides est réalisée à une température comprise entre 30 et 60°C.

9. Procédé selon l'une des revendications précédentes, dans lequel l'étape de régénération est réalisée à une pression comprise entre 1 bar et 10 bars et à une température comprise entre 100°C et 180°C.

10. Procédé selon l'une des revendications précédentes, dans lequel on effectue, avant l'étape de régénération, une première étape de détente de la solution absorbante chargée en composés acides.

11. Procédé selon l'une des revendications précédentes, dans lequel l'étape d'absorption est suivie d'au moins une étape de séparation liquide-liquide par chauffage de la solution absorbante chargée en composés acides, puis d'au moins une étape de régénération de la solution absorbante chargée en composés acides.

12. Procédé de traitement du gaz naturel selon l'une des revendications précédentes.

13. Procédé de traitement des gaz d'origine industrielle selon l'une des revendications précédentes.

14. Procédé de traitement des gaz d'origine industrielle selon la revendication 13 pour le captage du $CO_2$.

**Patentansprüche**

1. Verfahren zur Eliminierung von sauren Verbindungen enthalten in einem gasförmigen Abfluss in dem:

- man einen Schritt der Absorption der sauren Verbindungen durchführt, indem der Abstrom mit einer Absorptionslösung derart in Kontakt gebracht wird, um einen gasförmigen Abstrom zu erhalten, der an sauren Verbindungen verarmt ist und eine Absorptionslösung zu erhalten, die mit sauren Verbindungen angereichert ist, wobei die Absorptionslösung umfasst:

a. Wasser
b. wenigstens ein Amin der Formel (I), (N,N,N',N'-Tetramethylhexan-1,6-Diamin)

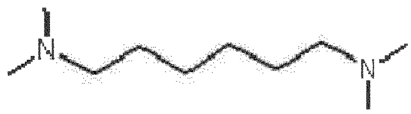

c. wenigstens eine Aktivierungsverbindung ausgesucht aus dem N-Butylpiperazin und einer Verbindung, die der folgenden Formel (II) entspricht

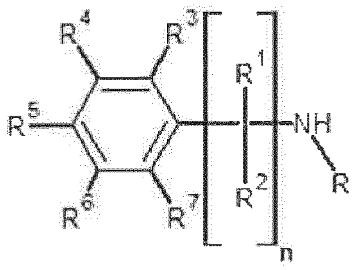

bei der n=1 oder 2 ist,
und bei der jeder der Reste R1, R2, R3, R4, R5, R6, R7 und R unabhängig voneinander ausgewählt ist aus den Elementen der Gruppe gebildet durch: ein Wasserstoffatom, eine Alkylgruppe mit einem bis zwei Kohlenstoffatomen,
und in der die Gruppe R eine der folgenden Charakteristika besitzt:

die Gruppe R ist nicht verbunden mit einer der anderen Gruppen R1 bis R7,
die Gruppe R ist über R3 oder R7 derart an den aromatischen Ring der Formel (II) gebunden, dass sich ein Heterozyklus mit 5 bis 6 Atomen ergibt, und

- man einen Schritt der Regeneration durchführt, in dem man wenigstens einen Teil der mit sauren Verbindungen beladenen Lösung in eine Destillationskolonne schickt, um die sauren Verbindungen in Form eines gasförmigen Abstroms freizusetzen und eine regenerierte Absorptionslösung zu erhalten.

**2.** Verfahren nach Anspruch 1, in dem die Absorptionslösung zwischen 10 und 90 Gew.-% von N,N,N',N' Tetramethylhexan-1,6-Diamin enthält.

**3.** Verfahren nach einem der Ansprüche 1 und 2, in dem die Absorptionslösung eine Menge des oder der Aktivierungsverbindungen enthält, die von Null verschieden ist und kleiner als 50 Gew. % beträgt.

**4.** Verfahren nach einem der Ansprüche 1 bis 3, in dem der oder die Aktivierungsverbindungen ausgewählt sind aus der Gruppe gebildet durch:

- Benzylamin,
- N-Methylbenzylamin,
- N-Ethylbenzylamin,
- α-MethylBenzylamin,
- α-EthylBenzylamin,
- Phenethylamin,
- Tetra-Hydro-Iso-Quinolin,
- Iso-Indolin,
- N-Butylpiperazin.

**5.** Verfahren nach einem der Ansprüche 1 bis 4, in dem die Absorptionslösung ein physikalisches Lösungsmittel enthält.

**6.** Verfahren nach einem der Ansprüche 1 bis 5, in dem die Absorptionslösung eine organische oder anorganische Säure enthält.

**7.** Verfahren nach einem der vorhergehenden Ansprüche, in dem der Schritt der Absorption der sauren Verbindungen durchgeführt wird bei einem Druck zwischen 1 Bar und 120 Bar und bei einer Temperatur zwischen 30° und 100°C.

**8.** Verfahren nach einem der vorhergehenden Ansprüche, bei dem der Schritt der Absorption der sauren Verbindungen bei einer Temperatur zwischen 30° und 60°C durchgeführt wird.

**9.** Verfahren gemäß einem der vorhergehenden Ansprüche, bei dem der Regenerationsschritt bei einem Druck zwischen 1 Bar und 10 Bar und bei einer Temperatur zwischen 100°C und 180°C durchgeführt wird.

**10.** Verfahren nach einem der vorhergehenden Ansprüche, bei dem man vor dem Schritt der Regeneration einen ersten Schritt der Entspannung der mit sauren Verbindungen beladenen Absorptionslösung durchführt.

**11.** Verfahren nach einem der vorhergehenden Ansprüche, bei dem der Schritt der Absorption gefolgt wird von wenigstens einem Schritt der Flüssig- Flüssig-Trennung durch Erwärmen der mit sauren Verbindungen beladenen Absorptionslösung, dann von wenigstens einer Stufe der Regeneration der mit sauren Verbindungen beladenen Absorptionslösung.

**12.** Verfahren zur Behandlung von Erdgas gemäß einem der vorhergehenden Ansprüche.

**13.** Verfahren zur Behandlung von industriellen Gasen gemäß einem der vorhergehenden Ansprüche.

**14.** Verfahren zur Behandlung von industriellen Gasen gemäß Anspruch 13 zum Einfangen von $CO_2$.

## Claims

1. A method for removing acid compounds contained in a gaseous effluent, comprising:

 - carrying out an acid compound absorption stage by contacting the effluent with an absorbent solution so as to obtain a gaseous effluent depleted in acid compounds and an absorbent solution laden with acid compounds, the absorbent solution containing:

 a- water,
 b- at least one amine of formula (I) (N,N,N',N'-tetramethylhexane-1,6-diamine)

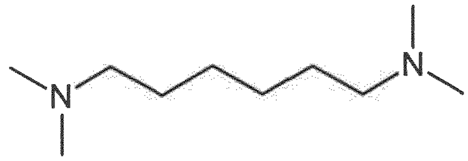

(I)

 c- at least one activating compound selected from among N-ButylPiperazine and a compound meeting formula (II)

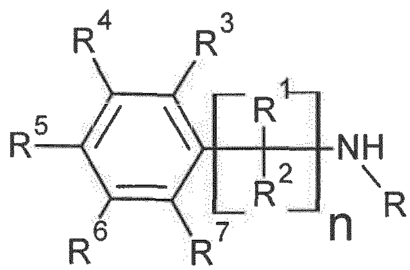

 wherein:

 n=1 or 2,
 each group R1, R2, R3, R4, R5, R6, R7 and R is selected independently from among one of the elements of the group made up of: a hydrogen atom, an alkyl group with 1 to 2 carbon atoms,
 and group R has one of the following characteristics:

 - group R is not linked to any one of groups R1 to R7,
 - group R is linked by R3 or R7 to the aromatic ring of formula (II) so as to form a heterocycle with 5 or 6 atoms, and
 - carrying out a regeneration stage wherein at least part of the solution laden with acid compounds is sent to a distillation column so as to release the acid compounds in form of a gaseous effluent and to obtain a regenerated absorbent solution.

2. A method as claimed in claim 1, wherein the absorbent solution comprises 10 to 90 wt % of N,N,N',N'-tetramethylhexane-1,6-diamine.

3. A method as claimed in any one of claims 1 and 2, wherein the absorbent solution comprises a non-zero proportion, below 50 wt %, of activating compound(s).

4. A method as claimed in any one of claims 1 to 3, wherein the activating compound(s) are selected from the group made up of:

 - BenzylAmine
 - N-MethylBenzylAmine
 - N-EthylBenzylAmine
 - α-MethylBenzylAmine

- α-EthylBenzylAmine
- PhenetylAmine
- TetraHydroIsoQuinoline
- IsoIndoline
- N-ButylPiperazine.

**5.** A method as claimed in any one of claims 1 to 4, wherein the absorbent solution comprises a physical solvent.

**6.** A method as claimed in any one of claims 1 to 5, wherein the absorbent solution comprises an organic or inorganic acid.

**7.** A method as claimed in any one of the previous claims, wherein the acid compound absorption stage is carried out at a pressure ranging between 1 and 120 bars, and at a temperature ranging between 30°C and 100°C.

**8.** A method as claimed in any one of the previous claims, wherein the acid compound absorption stage is carried out at a temperature ranging between 30°C and 60°C.

**9.** A method as claimed in any one of the previous claims, wherein the regeneration stage is carried out at a pressure ranging between 1 and 10 bars, and at a temperature ranging between 100°C and 180°C.

**10.** A method as claimed in any one of the previous claims, wherein a first stage of expansion of the absorbent solution laden with acid compounds is carried out prior to the regeneration stage.

**11.** A method as claimed in any one of the previous claims, wherein the absorption stage is followed by at least one liquid-liquid separation stage by heating the absorbent solution laden with acid compounds, then by at least one stage of regeneration of the absorbent solution laden with acid compounds.

**12.** A method for treating natural gas as claimed in any one of the previous claims.

**13.** A method for treating gases of industrial origin as claimed in any one of the previous claims.

**14.** A method for treating gases of industrial origin as claimed in claim 13 for $CO_2$ capture.

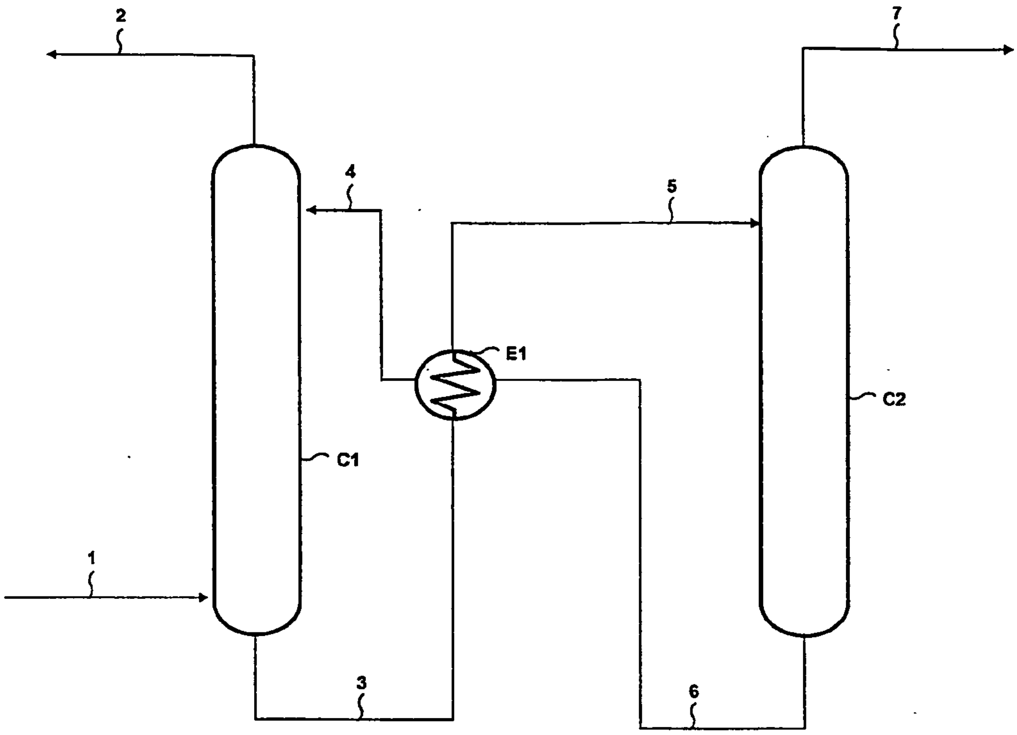


FIG. 1

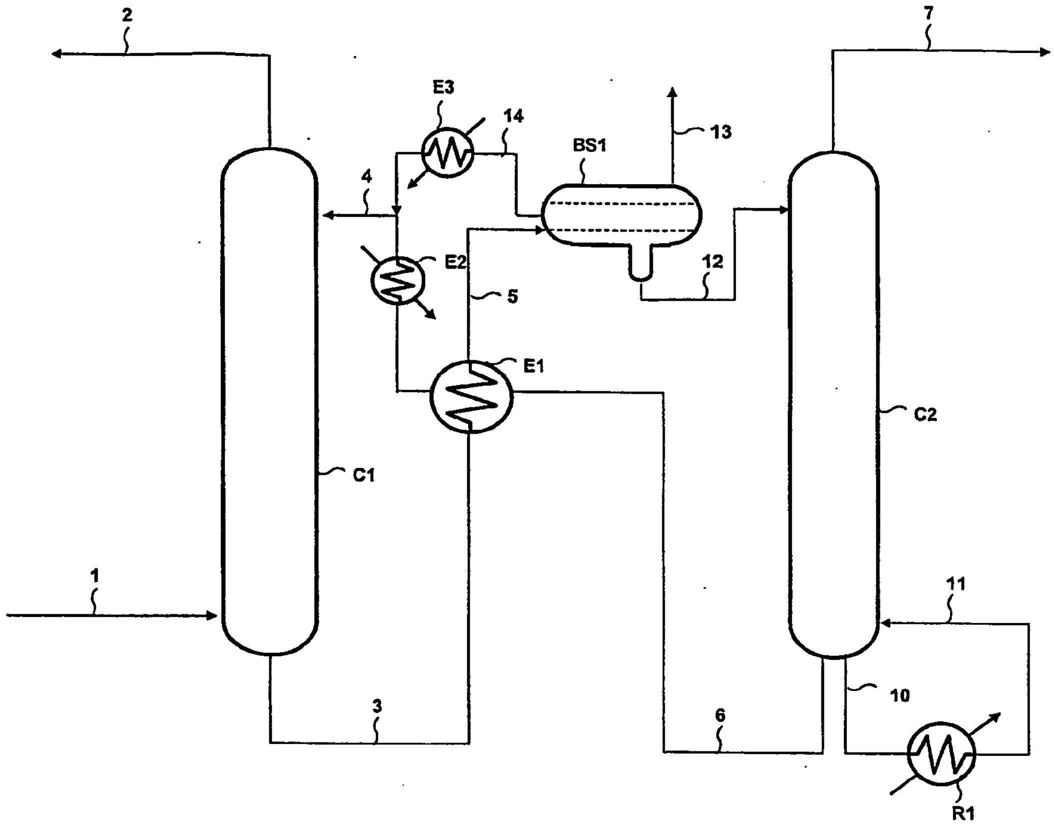


FIG. 2

## RÉFÉRENCES CITÉES DANS LA DESCRIPTION

*Cette liste de références citées par le demandeur vise uniquement à aider le lecteur et ne fait pas partie du document de brevet européen. Même si le plus grand soin a été accordé à sa conception, des erreurs ou des omissions ne peuvent être exclues et l'OEB décline toute responsabilité à cet égard.*

### Documents brevets cités dans la description

- FR 2820430
- US 6852144 B
- FR 2898284
- FR 2900841
- JP 10341556 A
- EP 1998105636 A
- JP 6286224 A
- JP 5025241 A
- EP 1993118476 A
- EP 1988309343 A
- JP 61124298 A
- JP 1985 A
- JP 147734 A
- DE 19853523074
- JP 58238221 A
- JP 58234589 A